(19) Europäisches Patentamt
European Patent Office
Office européen des brevets

(11) EP 2 172 199 A1

(12) EUROPEAN PATENT APPLICATION

(43) Date of publication:
07.04.2010 Bulletin 2010/14

(51) Int Cl.:
*A61K 31/428* (2006.01) *A61K 9/20* (2006.01)
*A61K 9/28* (2006.01)

(21) Application number: 09178277.1

(22) Date of filing: 25.07.2003

(84) Designated Contracting States:
AT BE BG CH CY CZ DE DK EE ES FI FR GB GR HU IE IT LI LU MC NL PT RO SE SI SK TR

(30) Priority: 25.07.2002 US 398427 P
25.07.2002 US 398447 P
28.08.2002 US 406609 P
18.06.2003 US 479387 P

(62) Document number(s) of the earlier application(s) in accordance with Art. 76 EPC:
03771898.8 / 1 536 791

(71) Applicant: Pharmacia Corporation
Peapack, NJ 07977 (US)

(72) Inventors:
- Amidon, Gregory, E.
Portage, MI 49024 (US)
- Ganorkar, Loksidh, D.
Kalamazoo, MI 49009 (US)
- Heimlich, John, M.
Portage, MI 49002 (US)
- Lee, Ernest, J.
Kalamazoo, MI 49009 (US)
- Martino, Alice, C.
Kalamazoo, MI 49009 (US)
- Noack, Robert, M.
Grand Rapids, MI 49506 (US)
- Reo, Joseph, P.
Kalamazoo, MI 49009 (US)
- Skoug, Connie, J.
Portage, MI 49024 (US)

(74) Representative: Summers, Victoria Clare
Pfizer Limited
European Patent Department
Ramsgate Road
Sandwich, Kent CT13 9NJ (GB)

Remarks:
This application was filed on 08-12-2009 as a divisional application to the application mentioned under INID code 62.

# (54) Sustained release tablet comprising pramipexole

(57) A sustained - release pharmaceutical composition in a form of an orally deliverable tablet comprising an active pharmaceutical agent having solubility not less than about 10mg/ml, dispersed in a matrix comprising a hydrophilic polymer and a starch having a tensile strength of at least about 0.15kNcm$^{-2}$ at a solid fraction representative of the tablet.

EP 2 172 199 A1

## Description

FIELD OF THE INVENTION

The present invention relates to tablet formulations, and more particularly to a sustained-release tablet composition for oral delivery of a water-soluble drug or prodrug.

BACKGROUND OF THE INVENTION

Many active pharmaceutical agents, including drugs and prodrugs, have been formulated as orally deliverable dosage forms providing sustained release (otherwise known as slow release or extended release) of such agents over a period of time effective to permit once daily administration. A well-known system for formulating such dosage forms involves a matrix comprising a hydrophilic polymer wherein the agent is dispersed; the agent is released over a period of time in the gastrointestinal tract upon dissolution or erosion ofthe matrix. Sustained-release dosage forms comprising such a matrix system are conveniently prepared as compressed tablets, described herein as "matrix tablets".

Drugs and prodrugs having relatively high solubility in water, for example a solubility of about 10 mg/ml or greater, present challenges to the formulator wishing to provide a sustained-release dosage form, and the higher the solubility the greater are the challenges. These challenges are well illustrated in the cases ofpramipexole dihydrochloride, which has a solubility in water of about 200 mg/ml, and reboxetine mesylate, which has a solubility in water of about 250 mg/ml.

Pramipexole (I) is a dopamine $D_2$ receptor agonist useful in treatment of Parkinson's disease. Pramipexole as its dihydrochloride salt is commercially available in the United States as Mirapex® tablets of Pharmacia & Upjohn. These are immediate-release tablets in 0.125 mg, 0.25 mg, 0.5 mg, 1.0 mg and 1.5 mg strengths, designed for oral administration of a single tablet three times per day to provide a daily dose of 0.375 to 4.5 mg. See Physicians' Desk Reference 57th edition (2003), 2768-2772. Doses herein are expressed in amounts of pramipexole dihydrochloride monohydrate unless otherwise specified; 1.0 mg pramipexole dihydrochloride monohydrate is equivalent to about 0.7 mg pramipexole base.

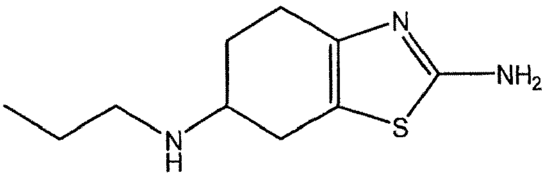

(I)

A three times daily dosing regimen for immediate-release pramipexole dihydrochloride tablets is well tolerated, but patient compliance would be much improved if a once-daily regimen were possible. In this regard, it will be noted that the primary indication for the drug, Parkinson's disease, is an affliction that becomes more prevalent with advancing age and is often accompanied by decline in memory. A once-daily regimen would be especially useful in enhancing compliance among elderly patients.

It has been found by the present inventors that formulation of pramipexole dihydrochloride monohydrate in a hydrophilic matrix tablet is generally inadequate to provide sustained-release properties consistent with once-daily dosing. Release characteristics can be further modified by coating the tablet with a sustained-release coating. Such a coating typically comprises a hydrophobic polymer and a hydrophilic pore-former.

The need to provide a coating over the matrix tablet gives rise to further problems. The additional handling operations involved in a coating step require a sufficient degree of tablet hardness to avoid tablet breakage and/or attrition during these operations, particularly in a high-speed manufacturing situation.

Reboxetine (II) is a selective noradrenaline reuptake inhibitor (SNRI) useful in treatment of depressive illness. Reboxetine as its methanesulfonate (mesylate) salt is commercially available in the United Kingdom and elsewhere as Edronax® tablets of Pharmacia & Upjohn. These are immediate-release tablets having a breaking score to facilitate division. Each Edronax® tablet contains 4 mg reboxetine and is designed for twice daily oral administration to provide a daily dose of 4 to 12 mg, with division of tablets if necessary. See British National Formulary 41st edition (2001), 196. Doses herein are expressed in amounts of reboxetine base unless otherwise specified.

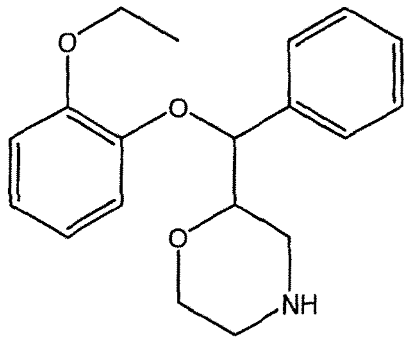

(II)

A twice-daily dosing regimen for immediate-release reboxetine tablets is well tolerated, but patient compliance would be much improved if a once-daily regimen were possible without substantially increasing the potential for adverse side effects. In this regard, it will be noted that the primary indication for the drug, depressive illness, is an affliction that is often accompanied by poor compliance.

It has been found by the present inventors that formulation of reboxetine salts in a hydrophilic matrix tablet can provide sustained-release properties consistent with once-daily dosing. However, the resulting tablets are susceptible to breakage and/or attrition during handling, especially in a high-speed tableting operation.

It has proved difficult to formulate a tablet having a suitable combination of sustained-release and handling properties, where the drug is one having relatively high solubility, as in the case of salts of pramipexole or reboxetine.

U.S. Patent No. 6,197,339 discloses a sustained-release tablet comprising (R)-5,6-dihydro-5-(methylamino)-4H-imidazo[4,5-ij]-quinolin-2(1H)-one (Z)-2-butenedioate (1:1) (sumanirole maleate) in a matrix comprising hydroxypropylmethylcellulose (HPMC) and starch. The tablet is disclosed to be useful in treatment of Parkinson's disease. Starches disclosed to be suitable therein include pregelatinized starch.

U.S. Patent No. 5,458,887 discloses a controlled-release tablet comprising an osmotic core that consists of a drug in admixture with a water-swellable component such as HPMC or polyethylene oxide, and a coating that comprises a water-resistant polymer and a minor amount of a water-soluble compound that acts as a pore-former. Upon formation of pores in the coating by dissolution ofthe water-soluble compound, the water-swellable agent is said to expand the core and provide a drug-rich surface in contact with gastrointestinal fluid.

U.S. Patent No. 5,656,296 discloses a dual control sustained-release formulation comprising a core that comprises a drug and a low melting point excipient, and a coating layer over the core that comprises a pH-independent water-insoluble polymer and a water-soluble film-forming polymer.

European Patent Application No. EP 0 933 079 discloses a starch said to be suitable for preparing tablets having high hardness yet being capable of rapid disintegration in an aqueous medium. Tensile strength of the finished tablets is calculated from the hardness.

Patents and publications cited above are incorporated herein by reference.

It is an object of the present invention to provide a sustained-release tablet composition of a water-soluble drug or prodrug that is suitable for once-daily oral administration. It is a further object to provide such a composition having sufficient hardness to withstand a high-speed tableting operation, in particular to resist erosion during application of a coating layer. It is a further object to provide a pharmaceutical tablet that provides day-long therapeutic effect on the central nervous system (CNS) of a subject when administered once daily. It is particular object to provide such a tablet that provides day-long therapeutic effect as a dopamine agonist when administered once daily, most particularly where the water-soluble drug is a salt of pramipexale, without substantially increased incidence of adverse side effects. It is another particular object to provide such a tablet that provides therapeutic effect as an SNRI when administered once daily, most particularly where the water-soluble drug is a salt of reboxetine or an enantiomer thereof, for example (S, S)-reboxetine, without substantially increased incidence of adverse side effects. It is a still further object to provide a method for testing a starch to assess its suitability for inclusion in a matrix tablet for sustained release of a water-soluble drug or prodrug.

SUMMARY OF THE INVENTION

There is now provided a sustained-release pharmaceutical composition in a form of an orally deliverable tablet comprising an active pharmaceutical agent having solubility not less than about 10 mg/ml, dispersed in a matrix comprising a hydrophilic polymer and a starch having a tensile strength of at least about 0.15 kN $cm^{-2}$ at a solid fraction representative of the tablet. The composition preferably exhibits sustained-release properties adequate to provide therapeutic effectiveness when administered orally not more than once daily to a subject in need thereof.

There is further provided a process for preparing a sustained-release pharmaceutical composition in a form of an orally deliverable tablet, the process comprising selecting by a suitable test a starch having a tensile strength of at least about 0.15 kN $cm^{-2}$ at a solid fraction representative of the tablet; admixing with the selected starch a hydrophilic polymer and an active pharmaceutical agent having solubility not less than about 10 mg/ml to provide a mixture wherein the agent is dispersed in a matrix comprising the polymer and the starch; and compressing the mixture to form a tablet.

A particularly convenient test method, which is itself a further embodiment of the invention, comprises preparing compacts of a starch sample on an automated tablet press at a range of compression forces, measuring hardness of the compacts, determining solid fraction of the compacts, calculating tensile strength of the compacts from hardness and dimensions of the compacts, determining relationship of tensile strength to solid fraction of the compacts, and from that relationship estimating tensile strength at a solid fraction representative of a desired tablet.

There is still further provided a method of treatment of a subject having a condition or disorder for which an active pharmaceutical agent having solubility not less than about 10 mg/ml is indicated, the method comprising orally administering to the subject a sustained-release pharmaceutical composition in a form of a tablet comprising the agent dispersed in a matrix comprising a hydrophilic polymer and a starch having a tensile strength of at least about 0.15 kN $cm^{-2}$ at a solid fraction representative of the tablet.

An "active pharmaceutical agent" herein can be a drug or a prodrug or a salt thereof, including diagnostic agents. Unless otherwise specified, "solubility" herein means solubility in water at 20-25°C at any physiologically acceptable pH, for example at any pH in the range of about 4 to about 8. In the case of an agent that is a salt, reference herein to solubility in water pertains to the salt, not to the free acid or base form ofthe agent.

The term "orally deliverable" herein means suitable for oral, including peroral and intra-oral (*e.g.*, sublingual or buccal) administration, but tablets of the present invention are adapted primarily for peroral administration, *i.e.*, for swallowing, typically whole or broken, with the aid of water or other drinkable fluid.

A "compact" herein is a compressed tablet, prepared for example on a tablet press, consisting only of a sample of starch for which it is desired to measure tensile strength. "Solid fraction" is the ratio of absolute to apparent density of a compact. A "solid fraction representative ofthe tablet" is a solid fraction selected to be similar to the solid fraction oftablets prepared according to the invention. Typically a solid fraction of about 0.75 to about 0.85, illustratively 0.8, will be selected.

A "subject" herein is an animal of any species, preferably mammalian, most preferably human. Conditions and disorders in a subject for which a particular agent is said herein to be "indicated" are not restricted to conditions and disorders for which the agent has been expressly approved by a regulatory authority, but also include other conditions and disorders known or believed by a physician to be amenable to treatment with the agent. "Treatment" herein embraces prophylactic treatment unless the context requires otherwise.

## BRIEF DESCRIPTION OF THE DRAWINGS

Fig. 1 is a graph showing relationship of tensile strength ofpregelatinized starch lots, as determined by a test method of the invention using a 4 second dwell time (Example 1 herein) to triaxial tensile strength.

Fig. 2 is a graph showing relationship of tensile strength ofpregelatinized starch lots, as determined by a test method of the invention using a 90 second dwell time (Example 1 herein) to triaxial tensile strength.

Fig. 3 is a graph showing correlation of tensile strength of pregelatinized starch lots with maximum hardness of tablets containing these lots.

Fig. 4 is a graph showing *in vitro* dissolution profiles of three different 0.375 mg sustained-release tablet formulations of pramipexole dihydrochloride monohydrate, as more fully described in Example 10.

Fig. 5 is a graph showing *in vitro* dissolution profiles of three different 4 mg sustained-release tablet formulations of (S,S)-reboxetine in the form of its succinate salt, as more fully described in Example 12.

## DETAILED DESCRIPTION OF THE INVENTION

The invention provides a pharmaceutical composition in a form of an orally deliverable tablet comprising a water-soluble active pharmaceutical agent. The composition preferably exhibits sustained-release properties adequate to provide therapeutic effectiveness when administered orally not more than once daily.

Typically, agents for which the invention is especially useful are unsuitable for once daily administration when formulated in an immediate-release composition. This unsuitability can arise from one or more properties of such agents including without limitation:

(a) short half-life ($T_{1/2}$) of the agent or active metabolite thereof in the bloodstream, requiring the plasma concentration to be "topped up" at intervals shorter than one day to maintain a therapeutically effective concentration; and

(b) potential for undesirable side effects arising from a high maximum plasma concentration ($C_{max}$) of the agent or therapeutically active metabolite thereof.

Relatively few agents having solubility not less than about 10 mg/ml are non-ionizable compounds. Most are compounds that exist as free acid or free base form and are present in such form, or more commonly in the form of a pharmaceutically acceptable salt, in a composition ofthe invention. Solubility of preferred agents is not less than about 50 mg/ml, more preferably not less than about 100 mg/ml. For purposes of the present invention, agents classified in the *United States Pharmacopeia*, 24th edition (2000) (USP 24) as "very soluble" or "freely soluble" are considered to have solubility not less than about 100 mg/ml, and together with agents classified in USP 24 as "soluble" or "sparingly soluble" are considered to have solubility not less than about 10 mg/ml.

Active pharmaceutical agents useful herein can be of any therapeutic category, for example any of the therapeutic categories listed in The Merck Index, 13th edition (2001). A partial list of agents useful herein is given below for illustration, noting that where one or more salts of an agent are listed, other pharmaceutically acceptable salts having solubility not less than about 10 mg/ml ("analogous salts") can be substituted:

abacavir sulfate
acarbose
acebutolol hydrochloride
acetylsalicylic acid calcium salt
acyclovir sodium
albuterol sulfate
alendronate sodium
alfentanil hydrochloride
almotriptan maleate
alosetron hydrochloride
amantadine hydrochloride
amdinocillin
aminolevulinic acid hydrochloride
aminophylline
*p*-aminasalicylate calcium, sodium and potassium salts
amitryptiline hydrochloride
amlodipine acetate, hydrochloride and mesylate salts
amphetamine phosphate and sulfate salts
arbutamine
atenolol
atropine sulfate
azlocillin sodium
balsalazide disodium
benazipril hydrochloride
benztropine mesylate
bethanechol chloride
bisoprolol fumarate
brompheniramine maleate
bupropion hydrochloride
caffeine and caffeine citrate
capecitabine
captopril
carbenicillin disodium
carbetapentane citrate
carbinoxamine maleate
cefaclor
cefmetazole sodium
cefodizime disodium
ceftezole sodium
ceftiofur sodium
ceftriaxone disodium
cefuroxime sodium
cefuzonam sodium

cetirizine hydrochloride
cevimeline hydrochloride
chlordiazepoxide hydrochloride
chloroquine phosphate
chlorpheniramine maleate
chlorpromazine hydrochloride
cilastatin sodium
cimetidine hydrochloride
clindamycin hydrochloride
clindamycin phosphate
clomipramine hydrochloride
clonidine hydrochloride
clorazepate dipotassium
codeine hydrochloride and sulfate salts
codeine phosphate
cyclobenzaprine hydrochloride
cyproheptadine hydrochloride
cysteamine hydrochloride
daunorubicin hydrochloride
desipramine hydrochloride
dexamethasone 21-phosphate disodium
dextromethorphan hydrobromide
dibekacin
diclofenac potassium
dicloxacillin sodium
dicyclomine hydrochloride
didanosine
dihydrocodeine
diltiazem hydrochloride
diphenhydramine hydrochloride
disulfiram
dolasetron mesylate
donepezil hydrochloride
dopamine hydrochloride
dorzolamide hydrochloride
doxepin hydrochloride
doxycycline hyclate
eletriptan hemisulfate
enalapril maleate
epinastine hydrochloride
erythromycin glucoheptonate and lactobionate salts
ethosuximide
etidronic acid and disodium salt
etoperidone hydrochloride
fadrozole hydrochloride
famciclovir
fentanyl citrate
flucytosine
fludarabine phosphate
fluoxetine hydrochloride
fluvastatin sodium
fosfomycin and fosfomycin tromethamine
fosfosal
fosinopril sodium
fosphenytoin disodium
frovatriptan succinate
gabapentin
gatifloxacin

glycopyrrolate
granisetron hydrochloride
guaifenesin
haloperidol hydrochloride
homatropine methylbromide
hydralazine hydrochloride
hydrocodone bitartrate and hydrochloride salts
hydromorphone hydrochloride
hydroxychloroquine sulfate
hydroxyzine dihydrochloride
hyoscyamine hydrobromide
imatinib mesylate
imipramine hydrochloride
incadronate disodium
indinavir sulfate
isoniazid
isoxsuprine hydrochloride
ketorolac tromethamine
labetalol hydrochloride
lamivudine
levamisole hydrochloride
levetiracetam
lidocaine hydrochloride
lisinopril
losartan potassium
mafenide acetate
mecamylamine hydrochloride
medetomidine hydrochloride
meglutol
meperidine hydrochloride
metaraminol bitartrate
metformin hydrochloride,
methadone hydrochloride
methamphetamine hydrochloride
methenamine
methimazole
methscopolamine bromide
methyldopa
methylphenidate hydrochloride
methylprednisolone 21-succinate sodium
metoclopramide
metoprolol succinate
metralindole hydrochloride
mexiletine hydrochloride
mezlocillin sodium
midazolam hydrochloride
midodrine hydrochloride
miglitol
mizaribine
moexipril hydrochloride
molindone hydrochloride,
montelukast sodium
morphine hydrochloride
morphine sulfate
morpholine salicylate
nalmefene hydrochloride
naloxone hydrochloride
naproxen sodium

naratriptan hydrochloride
nedocromil disodium
neostigmine bromide and methylsulfate salts
nicotine bitartrate, salicylate and sulfate salts
nitrofurantoin
nizatidine
nortriptyline hydrochloride
ofloxacin
olsalazine sodium
ondansetron hydrochloride
orphenadrine hydrochloride
oxybutynin hydrochloride
oxycodone hydrochloride
pantoprazole sodium
parecoxib sodium
pemirolast potassium
penicillamine
penicillin G sodium and potassium salts
penicillin V potassium
pentamidine isethionate
pentobarbital sodium
pentosan polysulfate sodium
pentoxifylline
perindopril erbumine
phendimetrazine tartrate
phenelzine sulfate
phenoxybenzamine hydrochloride
phentermine hydrochloride
phenylephrine hydrochloride
phenylpropanolamine hydrochloride
phenytoin sodium
phenyltoloxamine citrate
pidotimod
pilocarpine hydrochloride
piperacillin sodium
pirenzepine dihydrochloride
pramipexole dihydrochloride
pravastatin sodium
prednisolone sodium phosphate
procainamide hydrochloride
procarbazine hydrochloride
procodazole
promethazine hydrochloride
propacetamol hydrochloride
propoxyphene hydrochloride
propranolol hydrochloride
protriptyline hydrochloride
pseudoephedrine hydrochloride
pyridostigmine bromide
quetiapine hemifumarate
quinapril hydrochloride
quinidine gluconate
quinine bisulfate
rabeprazole sodium
raltitrexed
ramosetron hydrochloride
ranitidine hydrochloride
reboxetine mesylate and succinate salts

ribavirin
rimantadine hydrochloride
risedronate sodium
rivastigmine tartrate
rizatriptan benzoate
ropinirole hydrochloride
scopolamine hydrobromide
selegiline hydrochloride
sotalol hydrochloride
stavudine
sulbenicillin disodium
sulfacetamide sodium
sumanirole maleate
sumatriptan succinate
tacrine hydrochloride
taurolidine
terazosin hydrochloride
tetracycline hydrochloride
theobromine sodium acetate and theobromine sodium salicylate
theophylline ethanolamine and theophylline isopropanolamine
theophylline sodium acetate and theophylline sodium glycinate
thioridazine hydrochloride
thyroxine sodium
ticlopidine hydrochloride
timolol maleate
tolterodine tartrate
tramadol hydrochloride
trientine hydrochloride
trifluoperazine dihydrochloride
valacyclovir hydrochloride
valganciclovir hydrochloride
valproate sodium
venlafaxine hydrochloride
verapamil hydrochloride
warfarin sodium
zolmitriptan
zolpidem hemitartrate

Other agents useful herein include N-[5-(1,4-diazepan-1-yl)-2-[(3-fluorophenyl)sulfonyl]phenyl]acetamide, N-[(3R)-1-azabicyclo[2.2.2]oct-3-yl]furo[2,3-c]pyridine-5-carboxamide and salts thereof

It will be understood that mention of an active pharmaceutical agent herein embraces racemates, enantiomers, polymorphs, hydrates and solvates thereof.

The present invention is especially suitable for highly potent drugs and prodrugs, *i.e.*, those that are therapeutically effective in low daily dosage amounts, for example not greater than about 100 mg/day, especially not greater than about 50 mg/day, more especially not greater than about 25 mg/day, even more especially not greater than about 10 mg/day, and most especially not greater than about 5 mg/day.

In one embodiment the active pharmaceutical agent has therapeutic effect on the central nervous system (CNS). Such agents, herein referred to as "CNS agents", are useful in treatment or prevention of CNS disorders, and include without limitation anticonvulsant, antidepressant, antidyskinetic, antiepileptic, antimanic, antimigraine, antimuscarinic, antiobsessional, antiparkinsonian, antipsychotic, antispasmodic, anxiolytic, cholinergic, CNS stimulant, dopamine receptor agonist, dopamine receptor antagonist, hypnotic, monoamine oxidase inhibitor, neuroleptic, neuroprotective, NMDA receptor antagonist, nootropic, prolactin inhibitor, sedative, selective serotonin reuptake inhibitor (SSRI), selective noradrenaline reuptake inhibitor (SNRI), serenic, serotonin receptor agonist, serotonin receptor antagonist and tranquilizer agents.

Illustrative CNS agents useful herein include salts of sumanirole, reboxetine, and pramipexole.

Sumanirole is used preferably in the form of its R-enantiomer, (R)-5,6-dihydro-5-(methylamino)-4H-imidazo[4,5-ij]-quinolin-2(1H)-one (III) and can be substituted by its thione counterpart (R)-5,6-dihydro-5-(methylamino)-4H-imidazo[4,5-ij]-quinoline-2(1H)-thione (IV).

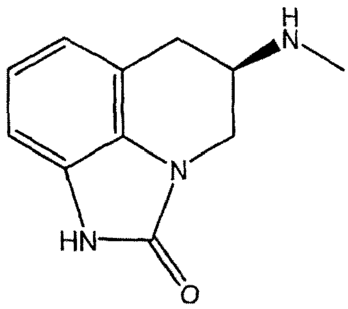
(III)

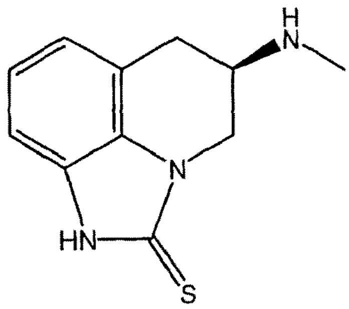
(IV)

In the case of either compound (III) or (TV), suitable salts include hydrochloride, hydrobromide, hydroiodide, sulfate, phosphate, acetate, propionate, lactate, maleate, malate, succinate, tartrate, cyclohexanesulfamate, mesylate (methanesulfonate), esylate (ethanesulfonate), besylate (benzenesulfonate) and tosylate (*p*-toluenesulfanate) salts. The maleate salt is preferred.

Sumanirole compositions of the invention are suitable for administration no more than twice daily, preferably no more than once daily. Such compositions are useful in treatment of any CNS condition or disorder for which sumanirole has therapeutic utility, but especially Parkinson's disease and complications associated therewith.

Reboxetine (II) can be used in the form of a racemic mixture comprising two or more of (R,R)-reboxetine, (S, S)-reboxetine, (R,S)-reboxetine and (S,R)-reboxetine, or in the form of any of these enantiomers alone. Preferably (S, S)-reboxetine is used.

Suitable salts ofreboxetine include hydrochloride, hydrobromide, hydroiodide, sulfate, phosphate, acetate, propionate, lactate, maleate, malate, succinate, fumarate, tartrate, cyclohexanesulfamate, mesylate, esylate, besylate and tosylate salts. In the case of a reboxetine racemate, the mesylate salt is preferred. In the case of (S,S)-reboxetine, the succinate and fumarate salts, more especially the succinate salt, are preferred.

Reboxetine and (S,S)-reboxetine compositions of the invention are preferably suitable for administration no more than once daily. Such compositions are useful in treatment of any CNS condition or disorder for which reboxetine and enantiomers thereof have therapeutic utility, but especially depressive illness and neuropathic pain, including postherpetic neuralgia and diabetic neuropathy.

Pramipexole (I) is used preferably in the form of its S-enantiomer, (S)-2-amino-4,5,6,7-tetrahydro-6-(propylamino)-benzothiazole. A preferred salt of pramipexole is the dihydrochloride salt, most preferably in the form ofthe monohydrate.

Pramipexole compositions of the invention are preferably suitable for administration no more than once daily. Such compositions are useful in treatment of any CNS condition or disorder for which pramipexole has therapeutic utility, but especially Parkinson's disease and complications associated therewith.

All active pharmaceutical agents useful herein can be prepared by processes known per se, including processes disclosed in patents and other literature pertaining to specific agents of interest.

The amount of the active pharmaceutical agent present in a composition of the invention depends on the potency of the agent, but is sufficient to provide a daily dose in one to a small plurality, for example one to about 4, of tablets to be administered at one time. Preferably the full daily dose is delivered in a single tablet. In most cases the amount ofthe agent per tablet is about 0.1 to about 200 mg, preferably about 0.2 to about 100 mg. Expressed as percentage by weight of the composition, the amount of the agent is typically about 0.0 1 % to about 25%, preferably about 0.05% to about 20%. In the case of an agent that is a salt, amounts of agent herein are expressed as free acid or free base equivalent amounts, unless otherwise specified.

Illustratively in the case of sumaniurole, an amount of about 0.5 to about 25 mg per tablet, or about 0.1% to about 15% by weight of the composition, will generally be suitable. Specific dosage amounts per tablet contemplated herein include 0.5, 1, 2, 4, 8, 12 and 24 mg sumanirole in the form of sumanirole maleate.

In the case of pramipexole, an amount of about 0.1 to about 10 mg per tablet, or about 0.05% to about 5% by weight ofthe composition, will generally be suitable. Preferably an amount of about 0.2 to about 6 mg, more preferably an amount of about 0.3 to about 5 mg, pramipexole per tablet is present. Specific dosage amounts per tablet contemplated herein include 0.375, 0.5, 0.75, 1.0, 1.5, 3.0 and 4.5 mg pramipexole dihydrochloride monohydrate.

In the case ofreboxetine or (S,S)-reboxetine, an amount of about 0.2 to about 15 mg per tablet, or about 0.1 % to about 10% by weight of the composition, will generally be suitable. Preferably an amount of about 1 to about 12 mg reboxetine or (S,S)-reboxetine per tablet is present. Specific dosage amounts per tablet contemplated herein include 1, 2, 4, 6, 8 and 12 mg reboxetine in the form of its mesylate salt or (S,S)-reboxetine in the form of its succinate salt.

A composition of the present invention comprises an active pharmaceutical agent as defined above, dispersed in a matrix comprising a hydrophilic polymer and a starch having a tensile strength of at least about 0.15 kN $cm^{-2}$ at a

solid fraction representative ofthe tablet, for example about 0.75 to about 0.85, illustratively 0.8.

Hydrophilic polymers useful herein are pharmaceutically acceptable polymeric materials having a sufficient number and distribution of hydrophilic substituents such as hydroxy and carboxy groups to impart hydrophilic properties to the polymer as a whole. Suitable hydrophilic polymers include, without limitation, methylcellulose, HPMC (hypromellose), carmellose (carboxymethylcellulose) sodium and carbomer (polyacrylic acid). More than one such polymer can optionally be used.

HPMC is a preferred hydrophilic polymer. Various types and grades of HPMC are available. In one embodiment HPMC type 2208, preferably meeting specifications set forth in a standard pharmacopeia such as USP 24, is used. HPMC type 2208 contains 19-24% by weight methoxy and 4-12% by weight hydroxypropoxy substituents. Especially suitable HPMCs have nominal viscosity ranging from about 100 to about 10,000 mPa s; illustratively a suitable HPMC type 2208 is one having a nominal viscosity of about 4,000, with a measured viscosity of about 3,000 to about 5,600 mPa s. Such an HPMC is available, for example, as Methocel® K4MP from Dow Chemical Co., and substantially equivalent products are available from other manufacturers.

The amount of hydrophilic polymer in the composition depends on the particular polymer selected, on the active pharmaceutical agent and on the desired sustained release profile. Typically, however, the hydrophilic polymer is included in an amount of about 20% to about 70%, preferably about 30% to about 60% and more preferably about 35% to about 50%, by weight of the composition. In the illustrative case of HPMC type 2208, a suitable amount will generally be found in the range from about 30% to about 60%, preferably about 35% to about 50%, for example about 40%, by weight of the composition.

It is believed, without being bound by theory, that the hydrophilic polymer functions to provide extended or sustained release of the active pharmaceutical agent, for example by gradual dissolution or erosion of the polymer in the gastrointestinal tract.

Starches useful herein include starches from any suitable botanical source, for example corn, wheat, rice, tapioca, potato, *etc.*. Preferred starches have a relatively high ratio of amylose to amylopectin, containing for example at least about 20%, more preferably at least about 25%, amylose. Especially preferred is pregelatinized starch, which is a type of modified starch that has been processed to render the starch more flowable and directly compressible. Partially or wholly pregelatinized starches can be used.

It is believed, without being bound by theory, that the primary function of the starch in a composition of the invention is as a binding agent. A starch meeting the tensile strength criterion defined herein can be referred to as a "super binder".

The amount of starch in the composition is typically higher than is conventionally present as a binder in tablet formulations. Suitable amounts will generally be found in the range of about 25% to about 75% by weight. Preferably the amount of starch is about 40% to about 70%, more preferably about 45% to about 65%, for example about 50%, by weight of the composition.

Tensile strength of a starch sample can be measured by any suitable test. Illustrative test procedures are described by Hiestand & Smith (1984), Powder Technology 38, 145-159, and by Hiestand & Smith (1991), International Journal of Pharmaceutics 67, 231-246, these articles being incorporated herein by reference.

An example of a tensile strength test that can be used (herein referred to as a "triaxial tensile strength test") requires preparation of a series of compacts ofthe starch sample, followed by determination of tensile strength of the compacts using a computerized multifunction tablet tester (MTT). The compacts are prepared with various degrees of compression force to provide compacts having a range of solid fraction. As a sustained release tablet formulation typically has a solid fraction of about 0.8, it is useful to prepare compacts approximating such a solid fraction.

Absolute density of the starch sample can be determined using a helium-air pycnometer.

A computer-controlled triaxial tablet press is used to prepare the compacts. Voltage output from the punch and die load cells of the tablet press are first zeroed. The punch and die are lubricated with magnesium stearate powder and the die assembly is placed in the press. Compression and decompression parameters are selected on the computer. The desired amount of starch to be compacted is weighed and poured into the die cavity. The resulting powder bed is leveled with a spatula. The punch is inserted into the die and the computer-controlled compression/decompression cycle is started.

Just prior to the end of the compression phase, thickness of the compact as measured by LVDT is recorded. At the end ofthe compression phase, the final compression force as measured by voltage of the punch load cell is recorded.

At the end of the decompression phase, the punch and die rams are retracted. The compact is removed from the die and inspected for defects, such as cracking or sticking. Cracking can be reduced by increasing decompression time. If the compact is free of defects, its length, width, thickness and weight are measured to enable calculation of apparent density. Solid fraction is calculated by dividing absolute density by apparent density.

In preparation of the MTT for tensile strength determination, a suitable software program is run. The platen is screwed to the load cell of the MTT and the tensile strength assembly is slid into the MTT opposite the platen. The load cell signal is monitored via the computer and the zero offset on the signal conditioner is adjusted to provide a positive

baseline voltage as close as possible to zero. A forward velocity is selected that will generate a time constant of approximately 15 seconds (usually the velocity selected will be about 0.8 to about 1.2 mm $s^{-1}$).

The compact to be tested is placed in the holder of the tensile strength assembly. The motor is initiated via the computer, driving the platen toward the compact until the surface ofthe compact is detected, and stopping the platen a few millimeters from the compact. The oscilloscope is triggered, to record the force applied to the compact, and the motor is restarted. The platen is driven into the compact until a crack is detected, either by sight or by sound, and the motor is immediately reversed.

Peak force is recorded from the oscilloscope trace. Tensile strength is calculated from the peak force using appropriate computer software.

From several runs using compacts at a range of solid fractions around 0.8, data are plotted and tensile strength at a solid fraction of 0.8 is estimated. If the tensile strength at a solid fraction of 0.8 is about 0.15 kN $cm^{-2}$ or greater, the starch sample is deemed to be suitable for use in preparing a composition according to the invention.

It has now surprisingly been discovered that a much simpler test, one that is more amenable to implementation in a manufacturing setting, can be used to estimate tensile strength of a starch sample, in particular to determine whether the starch sample has a tensile strength of at least about 0.15 kN $cm^{-2}$ at a solid fraction representative of a desired sustained-release tablet.

According to this test, compacts of the starch sample are prepared on a standard automated tablet press under a range of compression forces. For example, a Carver press (*e.g.*, Model 3888.1DT0000) fitted with flat-faced tooling of suitable diameter (*e.g.*, 10/32 inch or about 0.7 cm for a 300 mg compact), operated at compression forces of about 4 to about 16 kN (about 900 to about 3600 1bf) for a dwell time of at least about 4 seconds has been found to give satisfactory results. Illustratively, such compacts can be prepared at 1000, 1500, 2000 and 3000 lbf (4.45, 6.67, 8.90 and 13.34 kN). Preferably a dwell time of at least about 10 seconds, more preferably at least about 30 seconds, still more preferably at least about 60 seconds, is used. Illustratively, a dwell time of 90 seconds has been found to give satisfactory results. Weight, diameter and thickness of each compact are measured accurately (alternatively, diameter can be assumed to equal that of the tooling) to enable calculation of apparent density and hence solid fraction, absolute density having been measured as described above, for example by helium-air pycnometry.

Hardness of each compact thus prepared is then determined by any suitable tablet hardness test, for example using a Key HT 500 hardness tester. Hardness is a measure of the force required to cause crushing of the compact, and is typically expressed in units such as kiloponds (kp) or Strong-Cobb units (SCU). A hardness of about 10.2 kp or about 14.4 SCU corresponds to a force of 0.1 kN.

For present purposes it is considered that crushing strength of the compact is equivalent to tensile strength. Thus tensile strength ($\sigma_T$, in kN $cm^{-2}$) can be calculated from the equation

$$\sigma_T = 2F/\pi DH$$

where F is the force required to cause crushing (in kN), D is diameter of the compact (in cm) and H is thickness of the compact (in cm). For example, a compact of diameter 0.7 cm and thickness 0.4 cm having a hardness of 20 SCU (equivalent to a force of 0.139 kN) has a calculated tensile strength af 0.316 kN $cm^{-2}$.

The relationship between tensile strength and solid fraction is next established for the starch sample. This can be done by plotting data for tensile strength and solid fraction on a graph (solid fraction tends to increase with increasing compression force during preparation of the compact) or by performing a regression analysis. From that relationship, tensile strength at a standardized value of solid fraction can be estimated. The standardized value selected is one that is representative of the solid fraction of a desired sustained-release tablet, *e.g.*, 0.8.

Where the material ofthe compact is pregelatinized starch, it has been found that tensile strength as determined in a simple test as described immediately above is surprisingly close to a "true" tensile strength measurement as determined by the triaxial tensile strength test method previously described, which in turn is essentially similar to methods known in the art such as that disclosed by Hiestand & Smith (1984), *op. cit.*

It has also been found that a longer dwell time (*e.g.*, 90 seconds) in the test method of the present invention gives a better correlation with triaxial tensile strength than a very short dwell time (*e.g.*, 4 seconds). See Example 1 below and Figs. 1 and 2.

Thus, according to one embodiment of the invention, a method is provided for determining suitability of a starch for use in a sustained-release orally deliverable tablet comprising an active pharmaceutical agent having solubility not less than about 10 mg/ml, dispersed in a matrix comprising a hydrophilic polymer and a starch. The method comprises the steps of

(a) preparing compacts of a starch sample on an automated tablet press at a range of compression forces applied

for a dwell time of at least about 4 seconds;
(b) measuring hardness of each compact, expressed as the force required to cause crushing of the compact;
(c) determining solid fraction of each compact;
(d) calculating tensile strength $\sigma_T$ of each compact from the equation

$$\sigma_T = 2F/\pi DH$$

where F is the force required to cause crushing, D is diameter of the compact and H is thickness of the compact;
(e) establishing relationship of tensile strength to solid fraction of the compacts, for example by plotting these parameters on a graph and/or by performing a regression analysis; and
(f) using the relationship established in step (e), estimating tensile strength at a solid fraction representative of a desired sustained-release tablet; for example a solid fraction of 0.8.

If tensile strength of the starch as so estimated is at least about 0.15 kN $cm^{-2}$, the starch is deemed suitable for use.

An especially preferred starch has a tensile strength of at least about 0.175 kN $cm^{-2}$, even more preferably at least about 0.2 kN $cm^{-2}$, at a solid fraction representative of a desired sustained-release tablet.

Even among commercially available pregelatinized starches, the preferred type of starch for use in a composition of the invention, considerable variation exists in tensile strength. Pregelatinized starches not meeting the tensile strength criterion established herein are not readily identified without testing, for example by a method as disclosed above. Such pregelatinized starches are generally unsuitable for commercial-scale manufacture of a sustained-release matrix tablet formulation of a water-soluble drug or prodrug, because of a problem as set forth immediately below.

An uncoated tablet, or a tablet core prior to coating, comprising starch and a hydrophilic polymer acting as a matrix for a water-soluble drug or prodrug requires to have a certain minimum hardness in order to be able to resist breakage and/or attrition due to mechanical stresses imposed during a high-speed tableting operation (including all steps up to and including filling of the tablets into containers). The minimum acceptable hardness will depend on a number of factors, including the severity of the mechanical stresses, but is typically at least about 20 SCU, preferably at least about 22 SCU, more preferably at least about 24 SCU (about 17 kp).

Hardness can be increased by increasing the compression force applied by the tablet press, but only up to a certain level. At least in the case of tablets as described herein, above a certain compression force, further increases in compression force give little or no further increase in tablet hardness. There is, in other words, a maximum hardness achievable by compression of a particular starch/hydrophilic polymer/active agent composition. A starch providing a maximum hardness inadequate to withstand the mechanical stresses of a high-speed tableting operation is unsuitable for the present purpose. As shown in Fig. 3, certain pregelatinized starches have been found to provide a maximum hardness of 20 SCU or less; these are now identified as starches having low tensile strength (0.1 kN $cm^{-2}$ or less according to the test method of the invention utilizing a dwell time of 90 seconds).

Even if a maximum hardness of at least about 20 SCU is achievable, with a starch of low tensile strength it may be achievable only by use of extremely high compression forces. A requirement for such forces reduces speed and efficiency and increases cost of a tableting operation and is undesirable for these reasons.

Where tablets are to be subjected to an additional process step after compression, in particular a coating step, exposure to mechanical stresses is greatly increased. According to a preferred embodiment, therefore, the sustained-release tablet of the invention further comprises a coating.

For drugs and prodrugs of high water solubility as specified herein, a hydrophilic polymer matrix is often inadequate to provide sustained release of sufficiently long duration to permit once daily administration. It is believed that such drugs are readily leached out of the hydrophilic matrix when contacted by an aqueous medium such as gastrointestinal fluid. It is therefore desirable to further slow the process of drug release by providing a release-controlling coating around the tablet. Such a coating typically comprises a hydrophobic or water-insoluble polymer component such as ethylcellulose together with a hydrophilic or water-soluble pore-forming component such as HPMC.

Where a starch is used having a tensile strength of at least about 0.15 kN $cm^{-2}$, preferably at least about 0.175 kN $cm^{-2}$, more preferably at least about 0.2 kN $cm^{-2}$, at a solid fraction representative of the tablet (*e.g.*, about 0.75 to about 0.85), the composition is found to be especially suited to a high-speed tableting operation that includes a step of coating the tablet with a release-controlling layer.

Alternatives to ethylcellulose and HPMC as components of a release coating layer include other cellulosic polymers (*e.g.*, methylcellulose, hydroxypropylcellulose, hydroxyethylcellulose, carboxymethylcellulose sodium, cellulose esters such as cellulose acetate, *etc.*), polyvinyl acetate, polyvinyl pyrrolidone, polymers and copolymers of acrylic acid and methacrylic acid and esters thereof, polyethylene glycol, carrageenan and other gums, and the like.

A release-controlling layer, if present, typically constitutes about 1 % to about 15%, preferably about 2.5% to about 10%, by weight of the tablet as a whole. The hydrophobic or water-insoluble component, preferably comprising ethylcellulose, typically constitutes about 1% to about 10%, preferably about 2% to about 7%, by weight of the tablet as a whole. The pore-forming component, preferably comprising HPMC, is typically present in an amount of about 5% to about 50%, preferably about 10% to about 40%, by weight of the water-insoluble or hydrophobic component.

The coating, if present, can optionally contain additional pharmaceutically acceptable excipients such as plasticizers, dyes, *etc.*

Illustratively, a release-controlling layer in an amount of about 2.5% to about 5% by weight of the tablet core (*i.e.*, the tablet weight excluding the coating) comprises an ethylcellulose-based material (*e.g.*, Surclease® of Colorcon) and an HPMC-based pore-forming material (*e.g.*, Opadry® of Colorcon) in a weight ratio of about 3:1 to about 4:1.

A release-controlling layer or coating should be applied at as uniform a thickness as possible to provide optimum control of release rate of the active pharmaceutical agent.

Alternatively or in addition, the sustained-release tablet of the invention comprises a nonfunctional coating. A nonfunctional coating can comprise a polymer component, for example HPMC, optionally with other ingredients, for example one or more plasticizers, colorants, *etc.* The term "nonfunctional" in the present context means having no substantial effect on release properties of the tablet, and does not imply that the coating serves no useful purpose. For example, such a coating can impart a distinctive appearance to the tablet, provide protection against attrition during packaging and transportation, improve ease of swallowing, and/or have other benefits. A nonfunctional coating should be applied in an amount sufficient to provide complete coverage of the tablet. Typically an amount of about 1% to about 10%, more typically an amount of about 2.5% to about 5%, by weight of the tablet as a whole, will be found suitable.

Uncoated tablets and cores of coated tablets of the invention optionally contain one or more pharmaceutically acceptable excipients in addition to the starch and hydrophilic polymer components described above. Such excipients include without limitation glidants and lubricants. Other conventional excipients known in the art can also be included.

A glidant can be used to improve powder flow properties prior to and during tableting and to reduce caking. Suitable glidants include colloidal silicon dioxide, magnesium trisilicate, powdered cellulose, starch, talc, tribasic calcium phosphate and the like. In one embodiment, colloidal silicon dioxide is included as a glidant in an amount up to about 2%, preferably about 0.2% to about 0.6%, by weight ofthe tablet.

A lubricant can be used to enhance release of a tablet from apparatus on which it is formed, for example by preventing adherence to the face of an upper punch ("picking") or lower punch ("sticking"). Suitable lubricants include magnesium stearate, calcium stearate, canola oil, glyceryl palmitostearate, hydrogenated vegetable oil, magnesium oxide, mineral oil, poloxamer, polyethylene glycol, polyvinyl alcohol, sodium benzoate, sodium lauryl sulfate, sodium stearyl fumarate, stearic acid, talc, hydrogenated vegetable oil, zinc stearate and the like. In one embodiment, magnesium stearate is included as a lubricant in an amount of about 0.1% to about 1.5%, preferably about 0.3% to about 1%, by weight of the tablet.

Tablets can be of any suitable size and shape, for example round, oval, polygonal or pillow-shaped, and optionally bear nonfunctional surface markings. Especially in the case of coated tablets they are preferably designed to be swallowed whole and are therefore typically not provided with a breaking score. Tablets of the invention can be packaged in a container, accompanied by a package insert providing pertinent information such as, for example, dosage and administration information, contraindications, precautions, drug interactions and adverse reactions.

There is also provided a method of treatment of a subject having a condition or disorder for which an active pharmaceutical agent having solubility not less than about 10 mg/ml is indicated, the method comprising orally administering to the subject a sustained-release pharmaceutical composition in a form of a tablet comprising the agent dispersed in a matrix comprising a hydrophilic polymer and a starch having a tensile strength of at least about 0.15 kN $cm^{-2}$ at a solid fraction representative of the tablet. Preferably the composition is administered no more than once daily.

In one embodiment, the condition or disorder is a CNS condition or disorder and the agent is a CNS agent as defined herein. CNS conditions and disorders include those having a neurologic and/or a psychiatric component.

Illustrative CNS conditions and disorders treatable by the method of the invention include, for example, personality disorders including paranoid, schizoid, schizotypal, bipolar, histrionic, delusional, narcissistic, emotionally unstable, psychopathic and sociopathic personality disorders; habit and impulse disorders including pathological gambling, stealing, trichotillomania, *etc.*; obsessive-compulsive disorder; passive-aggressive disorder; acute and transient psychotic disorders; psychotic depression; schizoaffective disorder; hypochondria; cyclothymia; dysthymia; manic-depressive illness; major depressive disorder; treatment-resistant depression; adult and childhood onset schizophrenias; drug dependence including harmful use and abuse of, addiction to or dependence on opioids, narcotics, barbiturates, alcohol, benzodiazepines, amphetamines, cocaine, cannabinoids, hallucinogens, stimulants, nicotine (tobacco) and solvents; withdrawal states and mood and psychotic disorders related to such dependence; sexual dysfunction including hypoactive sexual desire disorder, sexual aversion or avoidance and erectile dysfunction; gender identity disorders; sexual preference disorders; general anxiety disorder; social anxiety disorder; mixed anxiety and depressive disorder; attention deficit hyperactivity disorder (ADHD) and depression and anxiety associated therewith; depression, anxiety, emotional dysregulation and behavioral disturbances associated with mental retardation; developmental disorders including autism, Asperger's syndrome and Rett's syndrome; childhood conduct and attachment disorders; premenstrual dysphoric disorder; postpartum depression; phobias including social phobias, agoraphobia and specific phobias related for example to hospitals, injections, venesection, *etc.*; posttraumatic stress disorder; dissociative disorder; Briquet's syndrome; affective disorders including depression, bipolar affective disorder and recurrent depressive disorder; organic mood, anxiety and emotionally labile disorders resulting for example from brain damage or dysfunction arising from head injury, intracranial masses, stroke, *etc.*; chronic fatigue; stress-induced psychotic episodes; dementia including presenile dementia, Pick's disease, vascular dementia, multi-infarct dementia, Alzheimer's disease, dementia associated with Creutzfeldt-Jakob disease and HIV-related dementia; other neurodegenerative disorders including Parkinson's disease and Huntington's disease; suicidal behavior; eating disorders including anorexia, bulimia and binge eating disorder; adjustment disorders; somatization disorder; somatoform autonomic dysfunction; somatoform pain disorder; panic attacks; panic disorder; amnesia; neuropathic pain; fibromyalgia; migraine; epilepsy; tinnitus; enuresis; sleep disorders including insomnia, hypersomnia, narcolepsy, nightmares and night terrors; delirium; postconcussion syndrome; multiple sclerosis; tremors; muscular spasms; restless legs syndrome; Lennox-Gastaut syndrome; motor and vocal tic disorders; Tourette's syndrome; supranuclear palsy; Shy-Drager syndrome; trigeminal neuralgia; Bell's palsy; motor neuron diseases such as amyotrophic lateral sclerosis; and psychosomatic and psychosocial conditions associated with non-CNS diseases such as diabetes, inflammatory disease, infertility, allergies, psoriasis, asthma, hypertension, overactive bladder, thyroid disorders, obesity, immune disorders and cancer.

In a particular embodiment, the condition or disorder is one that is responsive to dopamine $D_2$ receptor agonist therapy or to SNRI therapy, and the active pharmaceutical agent is a dopamine $D_2$ receptor agonist or SNRI or prodrug thereof. Presently preferred dopamine $D_2$ receptor agonists for use according to the method of the invention include salts ofpramipexole and sumanirole. Such dopamine $D_2$ receptor agonists are particularly useful in treatment of Parkinson's disease. Presently preferred SNRIs for use according to the method of the invention include salts of reboxetine and (S,S)-reboxetine. Such SNRIs are particularly useful in treatment of depressive illness and neuropathic pain, including postherpetic neuralgia and diabetic neuropathy.

Illustratively in the case of sumanirole, suitable daily dosage amounts include 0.5, 1, 2, 4, 8, 12 and 24 mg sumanirole in the form of sumanirole maleate. In the case of pramipexole, suitable daily dosage amounts include 0.375, 0.5, 0.75, 1.0, 1.5, 3.0 and 4.5 mg pramipexole dihydrochloride monohydrate. In the case of reboxetine or (S,S)-reboxetine, suitable daily dosage amounts include 1, 2, 4, 6, 8 and 12 mg reboxetine in the form of its mesylate salt or (S, S)-reboxetine in the form of its succinate salt.

In a further embodiment, a composition ofthe invention is administered in combination therapy with one or more additional drugs or prodrugs. The term "combination therapy" herein means a treatment regimen wherein the agent provided by the composition of the invention and a second agent are administered individually or together, sequentially or simultaneously, in such a way as to provide a beneficial effect from co-action of these therapeutic agents. Such beneficial effect can include, but is not limited to, pharmacokinetic or pharmacodynamic co-action of the therapeutic agents. Combination therapy can, for example, enable administration of a lower dose of one or both agents than would normally be administered during monotherapy, thus decreasing risk or incidence of adverse effects associated with higher doses. Alternatively, combination therapy can result in increased therapeutic effect at the normal dose of each agent in monotherapy. "Combination therapy" herein is not intended to encompass administration of two or more therapeutic agents as part of separate monotherapy regimens that incidentally and arbitrarily result in sequential or simultaneous treatment.

Compositions of the invention can be especially suited to combination therapies, particularly where the second agent is one that is, or can be, administered once daily. There are significant advantages in patient convenience and compliance where both components of a combination therapy can be administered at the same time and with the same frequency. This is especially true in the case of geriatric patients or those suffering memory impairment.

When administered simultaneously, the two components of the combination therapy can be administered in separate dosage forms or in coformulatian, *i.e.*, in a single dosage form. When administered sequentially or in separate dosage forms, the second agent can be administered by any suitable route and in any pharmaceutically acceptable dosage form, for example by a route and/or in a dosage form other than the present composition. In a preferred embodiment, both components of the combination therapy are formulated together in a single dosage form.

An illustrative combination therapy involves once daily administration of a composition of the invention comprising an SNRI, for example a salt of reboxetine or (S,S)-reboxetine, and once daily administration of an SSRI, for example fluoxetine, fluvoxamine, paroxetine or sertraline or a salt thereof. SNRI/SSRI combination therapies have been proposed, *e.g.*, for treatment-resistant depression as disclosed by Forbes & Rogers (2003), Progress in Neurology and Psychiatry 7(2), 10-14; according to the present invention both components of such a combination therapy can be administered once daily, with concomitant improvement in patient compliance.

EXAMPLES

Example 1

Tensile strength of six commercially obtained lots of pregelatinized starch was determined using the triaxial tensile strength test procedure described hereinabove. Data for tensile strength at a solid fraction of 0.8 are presented in Table 1.

**Table 1. Tensile strength of pregelatinized starch lots at a solid fraction of 0.8 (triaxial test procedure)**

| Lot | Tensile strength (kN $cm^{-2}$) |
|---|---|
| 1 | 0.323 |
| 2 | 0.220 |
| 3 | 0.074 |
| 4 | 0.119 |
| 5 | 0.287 |
| 6 | 0.236 |

A great variation in tensile strength of pregelatinized starches was observed, ranging from 0.074 to 0.323 kN $cm^{-2}$. Lots 3 and 4, exhibiting the lowest values of tensile strength, were from one manufacturer. Lots 1, 5 and 6, exhibiting the highest values of tensile strength, were from a second manufacturer. Lot 2, exhibiting an intermediate value of tensile strength, was from a third manufacturer.

Example 2

Tensile strength of the same six lots af pregelatinized starch was determined by the following simplified test procedure.

Compacts of each starch lot were prepared on a Carver press, Model 3888.1DT0000 fitted with 10/32 inch (0.7 cm) flat-faced tooling, at compression forces of 1000, 1500, 2000 and 3000 lbf(4.45, 6.67, 8.90 and 13.34 kN), for a dwell time of 4 seconds or 90 seconds. Compacts of an additional three lots of pregelatinized starch (Lots 7, 8 and 9), from the same manufacturer as Lots 3 and 4, were prepared using a dwell time of 90 seconds only. Weight and thickness of each compact was measured (diameter being equal to that of the tooling) to enable calculation of apparent density. Absolute density of each starch lot was measured by helium-air pycnometry. Solid fraction was calculated as the ratio of apparent to absolute density.

Hardness (force required to cause crushing) of each compact was determined using a Key HT 500 hardness tester. Tensile strength was calculated from this force and dimensions of the compact, using the equation

$$\sigma_T = 2F/\pi DH$$

as described hereinabove.

A regression analysis was performed to determine the relationship of tensile strength to solid fraction for each starch lot, and tensile strength at a standardized solid fraction of 0.8 was calculated. Data are presented in Table 2.

**Table 2. Tensile strength of pregelatinized starch lots at a solid fraction of 0.8 (simplified test procedure of the invention)**

| Lot | Tensile strength (kN $cm^{-2}$) | |
|---|---|---|
| | 4 s dwell time | 90 s dwell time |
| 1 | 0.310 | 0.306 |
| 2 | 0.227 | 0.191 |
| 3 | 0.092 | 0.085 |
| 4 | 0,134 | 0.096 |

(continued)

| Lot | Tensile strength (kN $cm^{-2}$) | |
|---|---|---|
| | 4 s dwell time | 90 s dwell time |
| 5 | 0.316 | 0.277 |
| 6 | 0.333 | 0.242 |
| 7 | n.d. | 0.087 |
| 8 | n.d. | 0.088 |
| 9 | n.d. | 0.172 |

Correlation of tensile strength as measured in the simplified test using a 4 second dwell time (this Example) with tensile strength as measured by the triaxial test procedure of Example 1 is shown graphically in Fig. 1.

Correlation of tensile strength as measured in the simplified test using a 90 second dwell time (this Example) with tensile strength as measured by the triaxial test procedure of Example 1 is shown graphically in Fig. 2.

Both dwell times exhibited a strong correlation, but the correlation was especially close where the simplified test used a 90 second dwell time. It is concluded that the simplified test as herein described can be used to estimate tensile strength of a starch lot for the purpose of predicting whether that starch lot will be suitable for preparing a sustained-release tablet formulation of the present invention.

Example 3

Sumanirole maleate sustained-release tablets were prepared having the compositions shown in Table 3. Tablet strength in mg is expressed as sumanirole base.

**Table 3. Composition of sumanirole maleate tablets of Example 3**

| Ingredient | Tablet strength (mg) | | | | | | | |
|---|---|---|---|---|---|---|---|---|
| | 0.5 | 1 | 2 | 4 | 8 | 8 | 12 | 24 |
| | Amount (% by weight) | | | | | | | |
| sumanirole maleate | 0.23 | 0.45 | 0.9 | 1.8 | 3.6 | 3.6 | 5.4 | 10.9 |
| HPMC type 2208, 4000 mPa s | 35.00 | 35.00 | 35.0 | 35.0 | 35.0 | 35.0 | 35.0 | 35.0 |
| pregelatinized starch | 63.87 | 63.65 | 63.2 | 62.3 | 60.5 | 60.0 | 58.2 | 52.5 |
| colloidal silicon dioxide | 0.40 | 0.40 | 0.4 | 0.4 | 0.4 | 0.4 | 0.4 | 0.4 |
| magnesium stearate | 0.50 | 0.50 | 0.5 | 0.5 | 0.5 | 1.0 | 1.0 | 1.0 |

All ingredients except the lubricant (magnesium stearate) were screened to remove lumps and were blended thoroughly in a low-shear mixer operating at 24 rpm for 10-30 minutes. The lubricant was then screened into the mixer and the materials were blended for a further 2-5 minutes. The resulting lubricated mixture was compressed into 350 mg pillow-shaped tablets using a Kilian S100 tableting machine.

Example 4

Tablets similar to those of Example 3 were prepared using pregelatinized starches of lots 1-6 as tested in Examples 1 and 2. Maximum hardness of the tablets obtainable with each pregelatinized starch lot was determined.

Maximum hardness was correlated with tensile strength of the pregelatinized starch lot used, as measured in the simplified test of Example 2 using a 90 second dwell time. Results are shown in Fig. 3. The correlation was substantially linear.

In subsequent tests, tablets of different hardness were used as cores for coating and were tested for resistance to erosion during a high-speed coating operation. Tablet cores having a hardness of at least about 24 SCU (about 17 kp) were found to have acceptable resistance to erosion. As shown in Fig. 3, this degree of hardness is achievable using pregelatinized starch having a tensile strength of at least about 0.175 kN $cm^{-2}$. Pregelatinized starches of Lots 3 and 4 were unsuitable, having tensile strength less than about 0.15 kN $cm^{-2}$ and providing tablets having a maximum hardness

no greater than about 20 SCU (about 14 kp).

Example 5

Pramipexole dihydrochloride sustained-release tablets were prepared having the compositions shown in Table 4.

**Table 4. Composition of pramipexole dihydrochloride tablets of Example 5**

| Ingredient | Amount (mg) | | | | | | | |
|---|---|---|---|---|---|---|---|---|
| pramipexole dihydrochloride monohydrate | 0.375 | 0.75 | 1.5 | 3.0 | 4.5 | 0.375 | 0.375 | 4.5 |
| HPMC type 2208, 4000 mPa s | 140.0 | 140.0 | 140.0 | 140.0 | 140.0 | 70.0 | 157.5 | 157.5 |
| pregelatinized starch | 206.5 | 206.1 | 205.4 | 203.9 | 202.4 | 101.5 | 189.0 | 184.9 |
| colloidal silicon dioxide | 1.4 | 1.4 | 1.4 | 1.4 | 1.4 | 1.4 | 1.4 | 1.4 |
| magnesium stearate | 1.75 | 1.75 | 1.75 | 1.75 | 1.75 | 1.75 | 1.75 | 1.75 |
| total | 350 | 350 | 350 | 350 | 350 | 175 | 350 | 350 |

The tablets were prepared by the procedure described in Example 3, using pregelatinized starch having a tensile strength of at least about 0.175 kN $cm^{-2}$.

Example 6

Coated sustained-release tablets of pramipexole dihydrochloride were prepared having the composition shown in Table 5.

**Table 5. Composition of coated tablets of Example 6**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 7.88 |
| HPMC-based coating material (Opadry®) | 2.63 |
| total coating | 10.5 |

Tablet cores were prepared exactly as in Example 5, using pregelatinized starch having a tensile strength of at least about 0.175 kN $cm^{-2}$. A coating solution was prepared as follows. Opadry® HPMC-based material in an amount of 6.004 g was added to 106.682 g water and mixed for 45 minutes to provide an HPMC mixture. Next, 72.045 g Surelease® ethylcellulose-based material was added to the HPMC mixture and mixed for an additional 30 minutes to provide a coating solution.

The coating solution was applied to the tablet cores in an amount providing a 3% weight gain. The resulting coated tablets were cured using a 12 inch (about 30 cm) Vector LCDS or 24 inch (about 60 cm) Thomas Accela-Coata coating pan for about 15 minutes at a bed temperature of at least about 70°C. After curing, temperature was ramped down over a period of about 8 minutes to an exhaust temperature of about 45°C.

Example 7

Coated sustained-release tablets of pramipexole dihydrochloride were prepared having the composition shown in Table 6.

**Table 6. Composition of coated tablets of Example 7**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 8.4 |
| HPMC-based coating material (Opadry®) | 2.1 |
| total coating | 10.5 |

Tablet cores were prepared exactly as in Example 5, using pregelatinized starch having a tensile strength of at least about 0.175 kN $cm^{-2}$. A coating solution was prepared as follows. Opadry® HPMC-based material in an amount of 4.801 g was added to 103.041 g water and mixed for 45 minutes to provide an HPMC mixture. Next, 76.819 g Surelease® ethylcellulose-based material was added to the HPMC mixture and mixed for an additional 30 minutes to provide a coating solution.

Coating to a 3% weight gain and curing of the coated tablets were performed exactly as in Example 6.

Example 8

Coated sustained-release tablets of pramipexole dihydrochloride were prepared having the composition shown in Table 7.

**Table 7. Composition of coated tablets of Example 8**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 13.13 |
| HPMC-based coating material (Opadry®) | 4.38 |
| total coating | 17.5 |

Tablet cores were prepared exactly as in Example 5, using pregelatinized starch having a tensile strength of at least about 0.175 kN $cm^{-2}$. A coating solution was prepared as follows. Opadry® HPMC-based material in an amount of 10.003 g was added to 177.737 g water and mixed for 45 minutes to provide an HPMC mixture. Next, 120.03 g Surelease® ethylcellulose-based material was added to the HPMC mixture and mixed for an additional 30 minutes to provide a coating solution.

Coating to a 3% weight gain and curing of the coated tablets were performed exactly as in Example 6. After this first curing step, coating was repeated to provide a total tablet weight gain of about 5%, followed by curing for about

15 minutes at a bed temperature of at least about 70°C. After curing, temperature was ramped down over a period of about 8 minutes to an exhaust temperature of about 45°C.

Example 9

Coated sustained-release tablets of pramipexole dihydrochloride were prepared having the composition shown in Table 8.

**Table 8. Composition of coated tablets of Example 9**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 14.0 |
| HPMC-based coating material (Opadry®) | 3.5 |
| total coating | 17.5 |

Tablet cores were prepared exactly as in Example 5, using pregelatinized starch having a tensile strength of at least about 0.175 kN $cm^{-2}$. A coating solution was prepared as follows. Opadry® HPMC-based material in an amount of 8.002 g was added to 171.735 g water and mixed for 45 minutes to provide an HPMC mixture. Next, 128.032 g Surelease® ethylcellulose-based material was added to the HPMC mixture and mixed for an additional 30 minutes to provide a coating solution.

Coating to a 5% total weight gain and curing of the coated tablets were performed exactly as in Example 8.

Example 10

Dissolution profiles of the 0.375 mg pramipexole dihydrochloride tablets of each of Examples 5, 6 and 9 were evaluated in a standard *in vitro* USP dissolution assay under the following conditions. USP apparatus 1 was used to stir a dissolution medium (900 ml of 0.05M phosphate buffer at a pH of 6.8) at a spindle rotation speed of 100 rpm and a temperature of 37°C.

Data are shown in Fig. 4. The uncoated tablet of Example 5 and the tablet of Example 6 having a 3% coating comprising 25% pore-former exhibited very similar overall dissolution profiles. On close inspection, however, it will be noticed that the uncoated tablet of Example 5 showed faster initial dissolution, such that at 1 hour and 2 hour sampling times the percent dissolved was greater, than in the case of the coated tablet of Example 6. For example, at 1 hour, the coated tablet of Example 6 showed only 11% dissolution, while the uncoated tablet of Example 5 showed 15% dissolution. Similarly, at 2 hours, the coated tablet of Example 6 showed no more than 20% dissolution, while the uncoated tablet of Example 5 showed 24% dissolution.

Dissolution of the tablet of Example 9 having a 5% coating comprising 20% pore-former exhibited a dissolution profile much slower than either the tablet of Example 5 or the tablet of Example 6.

Example 11

(S,S)-reboxetine succinate sustained-release tablets were prepared having the compositions shown in Table 9. It will be noted that each tablet comprises 5.5 mg (S,S)-reboxetine succinate, equivalent to 4 mg (S,S)-reboxetine base.

**Table 9. Composition of (S,S)-reboxetine succinate tablets of Example 11**

| Ingredient | Amount (mg) | | |
|---|---|---|---|
| (S,S)-reboxetine succinate | 5.5 | 5.5 | 5.5 |

(continued)

| Ingredient | Amount (mg) | | |
|---|---|---|---|
| HPMC type 2208, 4000 mPa s | 40.0 | 80.0 | 160.0 |
| pregelatinized starch | 53.5 | 112.5 | 230.5 |
| colloidal silicon dioxide | 0.5 | 1.0 | 2.0 |
| magnesium stearate | 0.5 | 1.0 | 2.0 |
| total | 100.0 | 200.0 | 400.0 |

The tablets were prepared by the procedure described in Example 3, using pregelatinized starch having a tensile strength of at least about 0.175 kN $cm^{-2}$.

Example 12

Dissolution profiles of the 4 mg (S,S)-reboxetine tablets of Example 11 were evaluated in a standard *in vitro* USP dissolution assay under the following conditions. USP apparatus 2 was used to stir a dissolution medium (I liter of 0.05M phosphate buffer at a pH of 6.8) at a paddle rotation speed of 50 rpm and a temperature of 37°C. The medium was then filtered and samples were analyzed via UV detection.

Data are shown in Fig. 5. The tablet having 100 mg total weight exhibited the fastest dissolution, and the tablet having 400 mg total weight the slowest. The tablet having 200 mg total weight was intermediate in dissolution rate.

## Claims

**1.** A sustained-release pharmaceutical composition in a form of an orally deliverable tablet comprising an active pharmaceutical having solubility not less than 10mg/ml, the active pharmaceutical ingredient being dispersed in a matrix comprising a hydrophilic polymer and a starch having a tensile strength of at least 0.15 kN $cm^{-2}$ at a solid fraction of 0.75 to 0.85 of the tablet.

**2.** The composition of Claim 1 wherein the active pharmaceutical ingredient is pramipexole, or a salt thereof.

**3.** The composition of Claim 1 or 2, wherein the starch has a tensile strength of at least 0.175 kN $cm^{-2}$, preferably a tensile strength of at least 0.2 kN $cm^{-2}$ at a solid fraction of 0.75 to 0.85 of the tablet.

**4.** The composition of any of Claims 1 to 3 wherein the starch is a pregelatinized starch.

**5.** The composition of any of the preceding claims wherein the starch is present in an amount of 25% to 75%, preferably 40% to 70%, more preferably 45% to about 65%, by weight.

**6.** The composition of any of the preceding claims wherein the hydrophilic polymer is selected from the group consisting of methylcellulose, hydroxypropylmethylcellulose, carmellose sodium and carbomer, more preferably hydroxypropylmethylcellulose.

**7.** The composition of any of the preceding claims wherein the hydrophilic polymer is present in an amount of 20% to 70%, preferably 30% to 60%, more preferably 35% to 50%, by weight.

**8.** The composition of any of the preceding claims, further comprising **a** coating on the tablet.

**9.** The composition of Claim 8 wherein said coating is a release-controlling layer.

**10.** The composition of Claim 9 wherein said release-controlling layer constitutes 1% to 15% by weight of the tablet.

**11.** The composition of Claim 8 wherein said coating is a nonfunctional coating.

**12.** A process for preparing a sustained-release pharmaceutical composition according to Claim 1 in a form of an orally

deliverable tablet, the process comprising selecting by a suitable test a starch having a tensile strength of at least 0.15 kN $cm^{-2}$ at a solid fraction of 0.75 to 0.85 of the tablet; admixing with the selected starch a hydrophilic polymer and an active pharmaceutical agent which is reboxetine or a salt thereof, to provide a mixture wherein the agent is dispersed in a matrix comprising the polymer and the starch; and compressing the mixture to form said tablet.

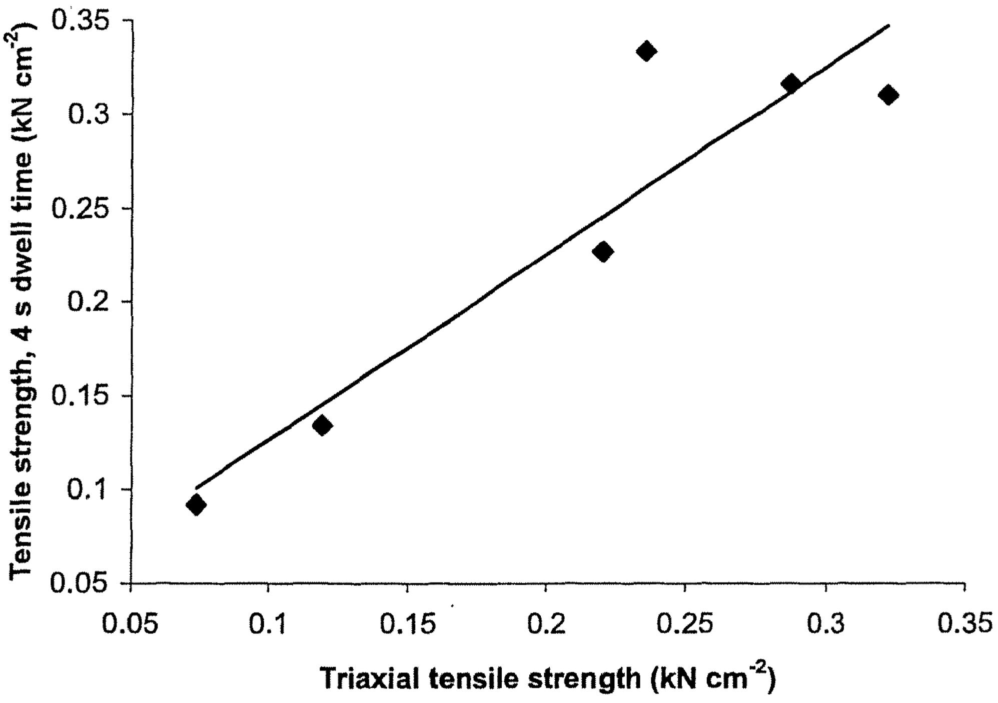
Tensile strength, 4 s dwell time (kN cm-2)
0.35
0.3
0.25
0.2
0.15
0.1
0.05
0.05
0.1
0.15
0.2
0.25
0.3
0.35
Triaxial tensile strength (kN cm-2)

Fig. 1

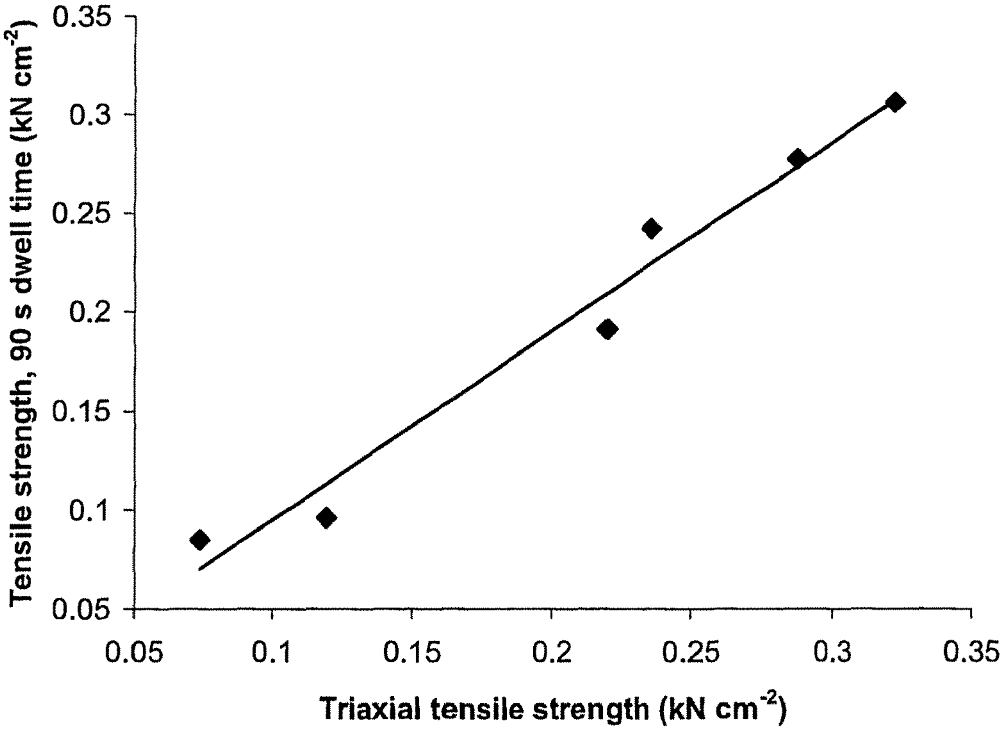
Tensile strength, 90 s dwell time (kN cm-2)
0.35
0.3
0.25
0.2
0.15
0.1
0.05
0.05
0.1
0.15
0.2
0.25
0.3
0.35
Triaxial tensile strength (kN cm-2)

Fig. 2

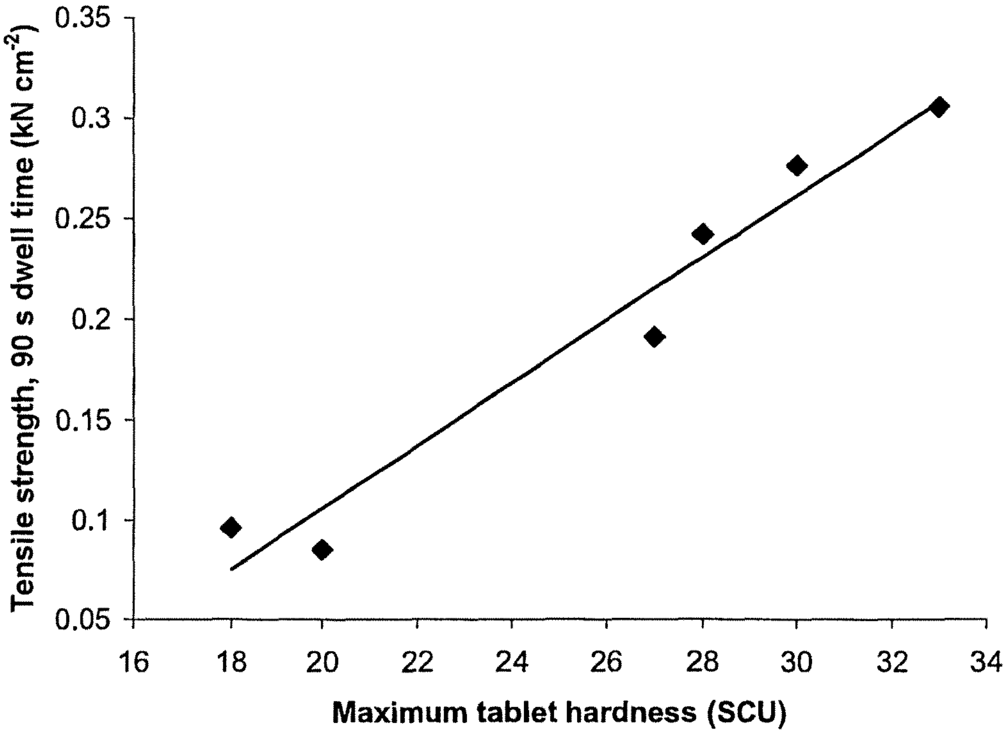
0.35
0.3
0.25
0.2
0.15
0.1
0.05
16
18
20
22
24
26
28
30
32
34
Tensile strength, 90 s dwell time (kN cm-2)
Maximum tablet hardness (SCU)

Fig. 3

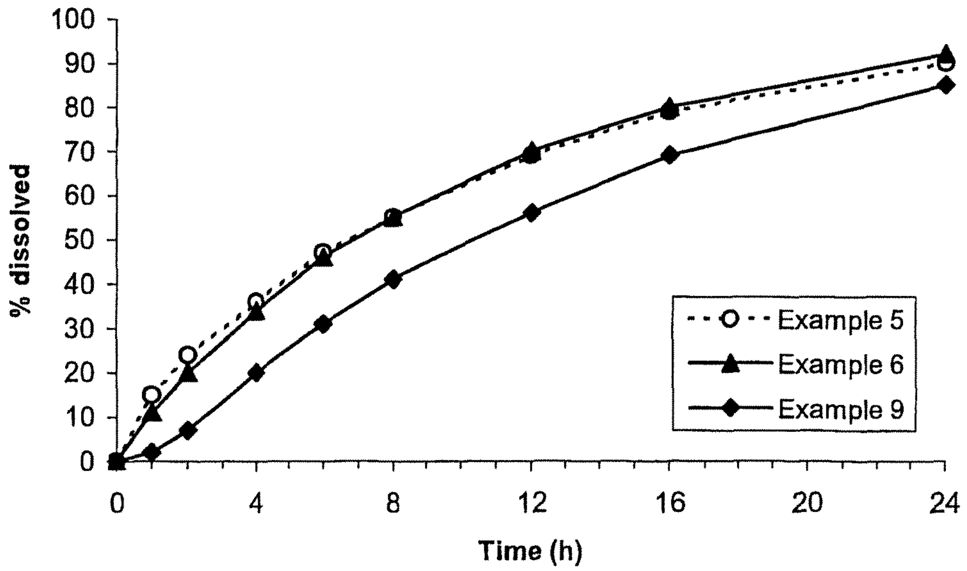
100
90
80
70
60
50
40
30
20
10
0
0
4
8
12
16
20
24
% dissolved
Time (h)
Example 5
Example 6
Example 9

Fig. 4

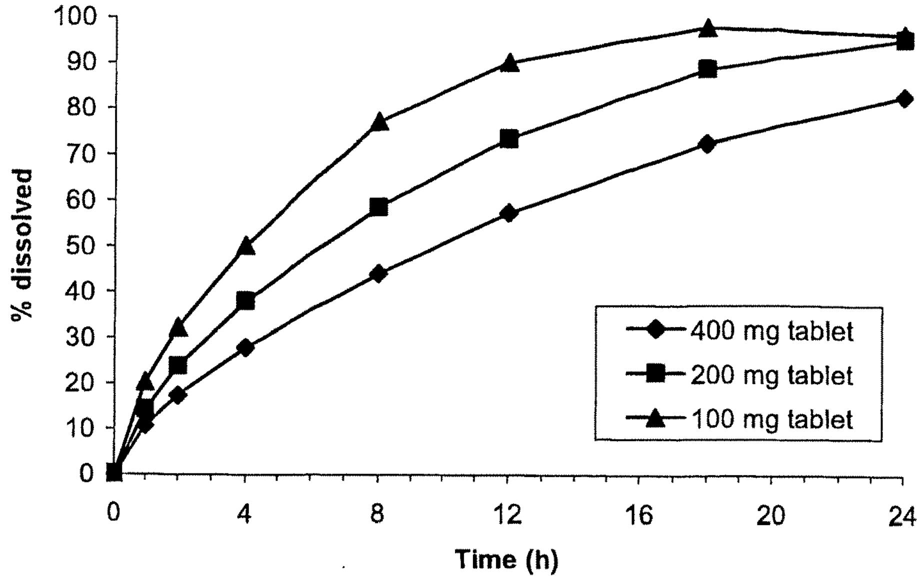
100
90
80
70
60
50
40
30
20
10
0
% dissolved
0
4
8
12
16
20
24
Time (h)
400 mg tablet
200 mg tablet
100 mg tablet

Fig. 5

Europäisches Patentamt
European Patent Office
Office européen des brevets

# EUROPEAN SEARCH REPORT

Application Number
EP 09 17 8277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 277 875 B1 (HOLMAN ANDREW J) 21 August 2001 (2001-08-21)<br>* column 7, line 32 - column 8, line 57 *<br>* column 11, line 20 - line 46 *<br>----- | 1-12 | INV.<br>A61K31/428<br>A61K9/20<br>A61K9/28 |
| Y | US 4 731 374 A (KOBINGER WALTER ET AL) 15 March 1988 (1988-03-15)<br>* column 7, line 16 - column 9, line 46 *<br>* examples I,V *<br>----- | 1-12 | |
| Y | WO 99/16442 A (JU TZU CHI ROBERT ;UPJOHN CO (US)) 8 April 1999 (1999-04-08)<br>* page 2, line 4 - line 13 *<br>* page 2, line 32 - line 36 *<br>* page 3, line 1 - line 11 *<br>* page 3, line 20 - line 29 *<br>* examples 9-11 *<br>----- | 1-12 | |
| Y | WO 00/59477 A (JANS EUGENE MARIE JOZEF ;JANSSEN PHARMACEUTICA NV (BE); VANDECRUYS) 12 October 2000 (2000-10-12)<br>* page 1, line 4 - line 12 *<br>* page 8, line 9 *<br>* page 10, line 26 - page 13, line 3 *<br>* page 20, line 21 - page 24, line 19 *<br>* examples *<br>----- | 1-12 | TECHNICAL FIELDS SEARCHED (IPC)<br>A61K |
| Y | WO 97/04752 A (DURAMED PHARMACEUTICALS INC) 13 February 1997 (1997-02-13)<br>* page 13, line 36 - page 14, line 8 *<br>* page 15, line 1 - page 16, line 7 *<br>* page 16, line 34 - page 17, line 29 *<br>* page 19, line 1 - line 15 *<br>* example 1 *<br>-----<br>-/-- | 1-12 | |

The present search report has been drawn up for all claims

3

| Place of search | Date of completion of the search | Examiner |
|---|---|---|
| Munich | 26 February 2010 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 17 8277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 99/09066 A (CARRIERE FRANCOIS ;DUMOULIN YVES (CA); ROUGIER INC (CA)) 25 February 1999 (1999-02-25) * page 11, line 5 - line 18 * * page 13, line 26 - line 32 * * examples 2B,4B,6B,8B * ----- | 1-12 | |
| A,D | EP 0 933 079 A (CERESTAR HOLDING BV) 4 August 1999 (1999-08-04) * examples * ----- | 1-12 | |

TECHNICAL FIELDS SEARCHED (IPC)

The present search report has been drawn up for all claims

| Place of search | Date of completion of the search | Examiner |
|---|---|---|
| Munich | 26 February 2010 | Giménez Miralles, J |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 17 8277

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2010

| Patent document cited in search report | Publication date | Patent family member(s) | Publication date |
|---|---|---|---|
| US 6277875 B1 | 21-08-2001 | AU 7191001 A | 30-01-2002 |
| | | AU 2001271910 B2 | 28-07-2005 |
| | | BR 0112476 A | 22-07-2003 |
| | | CA 2414774 A1 | 24-01-2002 |
| | | CN 1443066 A | 17-09-2003 |
| | | CZ 20030417 A3 | 13-08-2003 |
| | | EP 1311251 A2 | 21-05-2003 |
| | | HU 0301305 A2 | 28-08-2003 |
| | | JP 2004503587 T | 05-02-2004 |
| | | MX PA03000336 A | 19-04-2005 |
| | | NO 20030213 A | 11-03-2003 |
| | | NZ 523926 A | 26-05-2006 |
| | | PL 360121 A1 | 06-09-2004 |
| | | WO 0205797 A2 | 24-01-2002 |
| | | US 6300365 B1 | 09-10-2001 |
| | | ZA 200300588 A | 22-04-2004 |
| US 4731374 A | 15-03-1988 | AU 583874 B2 | 11-05-1989 |
| | | AU 5154485 A | 17-07-1986 |
| | | BG 62023 B2 | 30-12-1998 |
| | | CA 1263653 A1 | 05-12-1989 |
| | | CS 9104099 A3 | 16-09-1992 |
| | | DK 590285 A | 23-06-1986 |
| | | EP 0186087 A1 | 02-07-1986 |
| | | ES 8702787 A1 | 01-04-1987 |
| | | ES 8707513 A1 | 16-10-1987 |
| | | ES 8707514 A1 | 16-10-1987 |
| | | ES 8707515 A1 | 16-10-1987 |
| | | FI 855102 A | 23-06-1986 |
| | | GR 853126 A1 | 22-04-1986 |
| | | HK 78692 A | 23-10-1992 |
| | | HU 39736 A2 | 29-10-1986 |
| | | IE 58863 B1 | 17-11-1993 |
| | | IL 77415 A | 19-03-1990 |
| | | LU 90208 A9 | 06-04-1998 |
| | | NL 980002 I1 | 02-03-1998 |
| | | NO 855195 A | 23-06-1986 |
| | | NZ 214661 A | 26-04-1990 |
| | | PH 24533 A | 03-08-1990 |
| | | PT 81735 A | 01-01-1986 |
| | | SG 82492 G | 04-12-1992 |
| | | US 4843086 A | 27-06-1989 |
| | | US 4886812 A | 12-12-1989 |
| WO 9916442 A | 08-04-1999 | AT 297208 T | 15-06-2005 |
| | | AU 742941 B2 | 17-01-2002 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 09 17 8277

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2010

| Patent document cited in search report | Publication date | Patent family member(s) | Publication date |
|---|---|---|---|
| WO 9916442 A | | AU 9296498 A | 23-04-1999 |
| | | BR 9812687 A | 22-08-2000 |
| | | CA 2301869 A1 | 08-04-1999 |
| | | CN 1268890 A | 04-10-2000 |
| | | DE 69830503 D1 | 14-07-2005 |
| | | DE 69830503 T2 | 16-03-2006 |
| | | EP 1017391 A2 | 12-07-2000 |
| | | ES 2242296 T3 | 01-11-2005 |
| | | FI 20000720 A | 29-03-2000 |
| | | HK 1030163 A1 | 11-11-2005 |
| | | HU 0004586 A2 | 28-06-2001 |
| | | JP 2001517701 T | 09-10-2001 |
| | | NO 20001624 A | 29-03-2000 |
| | | NZ 504221 A | 26-11-2004 |
| | | PL 339946 A1 | 15-01-2001 |
| | | PT 1017391 E | 30-09-2005 |
| | | RU 2205007 C2 | 27-05-2003 |
| | | SK 3612000 A3 | 12-09-2000 |
| WO 0059477 A | 12-10-2000 | AT 313319 T | 15-01-2006 |
| | | AU 776645 B2 | 16-09-2004 |
| | | AU 3963800 A | 23-10-2000 |
| | | BG 65470 B1 | 30-09-2008 |
| | | BR 0009437 A | 15-01-2002 |
| | | CA 2371940 A1 | 12-10-2000 |
| | | CN 1345233 A | 17-04-2002 |
| | | CZ 20013375 A3 | 15-05-2002 |
| | | DE 60024981 T2 | 14-09-2006 |
| | | DK 1169024 T3 | 08-05-2006 |
| | | EE 200100505 A | 16-12-2002 |
| | | EP 1169024 A1 | 09-01-2002 |
| | | EP 1611881 A1 | 04-01-2006 |
| | | EP 1649851 A2 | 26-04-2006 |
| | | ES 2255490 T3 | 01-07-2006 |
| | | HK 1044715 A1 | 17-02-2006 |
| | | HR 20010700 A2 | 30-04-2003 |
| | | HU 0200611 A2 | 29-07-2002 |
| | | JP 2002541090 T | 03-12-2002 |
| | | KR 20060097068 A | 13-09-2006 |
| | | MX PA01009877 A | 06-05-2002 |
| | | NO 20014724 A | 28-09-2001 |
| | | NZ 514890 A | 30-05-2003 |
| | | PL 355096 A1 | 22-03-2004 |
| | | SI 1169024 T1 | 30-06-2006 |
| | | SK 13542001 A3 | 06-08-2002 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 09 17 8277

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2010

| Patent document cited in search report | Publication date | Patent family member(s) | Publication date |
|---|---|---|---|
| | | US 2009191266 A1 | 30-07-2009 |
| WO 0059477 A | | US 2004062805 A1 | 01-04-2004 |
| | | US 6667060 B1 | 23-12-2003 |
| WO 9704752 A | 13-02-1997 | AT 401062 T | 15-08-2008 |
| | | CA 2227887 A1 | 13-02-1997 |
| | | CN 1197387 A | 28-10-1998 |
| | | EP 0840599 A1 | 13-05-1998 |
| | | ES 2310415 T3 | 01-01-2009 |
| | | JP 4017664 B2 | 05-12-2007 |
| | | JP 2002504069 T | 05-02-2002 |
| | | TW 457092 B | 01-10-2001 |
| | | WO 9704753 A1 | 13-02-1997 |
| | | US 5908638 A | 01-06-1999 |
| WO 9909066 A | 25-02-1999 | AU 8724198 A | 08-03-1999 |
| | | CA 2211778 A1 | 14-02-1999 |
| EP 0933079 A | 04-08-1999 | AT 262321 T | 15-04-2004 |
| | | CA 2260637 A1 | 03-08-1999 |
| | | DE 69915718 D1 | 29-04-2004 |
| | | DE 69915718 T2 | 24-03-2005 |
| | | DK 933079 T3 | 26-07-2004 |
| | | ES 2216438 T3 | 16-10-2004 |
| | | JP 11269202 A | 05-10-1999 |
| | | US 6143324 A | 07-11-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6197339 B
- US 5458887 A
- US 5656296 A
- EP 0933079 A


### Non-patent literature cited in the description


- Physicians' Desk Reference. 2003, 2768-2772
- British National Formulary. 2001, 196
- The Merck Index. 2001
- **Hiestand ; Smith.** *Powder Technology,* 1984, vol. 38, 145-159
- **Hiestand ; Smith.** *International Journal of Pharmaceutics,* 1991, vol. 67, 231-246
- **Forbes ; Rogers.** *Progress in Neurology and Psychiatry,* 2003, vol. 7 (2), 10-14